# EUROPEAN PATENT APPLICATION

(11) **EP 0 707 067 A2**
(43) Date of publication of application: **17.04.1996**
(21) Application number: 95305675.1
(22) Date of filing: 15.08.1995
(51) Int. Cl.: C12N 15/57, C12N 9/64, A61K 38/48, C12Q 1/68

(54) **A novel gene coding thrombin-like protease**

(30) Priority: 11.10.1994 KR 9426001
(71) Applicant: Mogam Biotechnology Research Institute, Yongin-Kun, Kyonggi-Do (KR)
(72) Inventor: Kim, Hak-Dai, Yongin-Kun, Kyonggi-Do (KR); Yun, Yung-Dae, Songpa-Ku, Seoul (KR); Chung, Kwang-Hoe, Sungnam, Kyonggi-Do (KR); Kim, Doo-Sik, Seoul (KR); Moon, Hong-Mo, Pundang-Ku, Sungnam, Kyonggi-Do (KR)
(74) Representative: Woods, Geoffrey Corlett

(57) **Abstract**

The present invention relates to a novel cDNA sequence encoding a thrombin-like serine protease ("Halybin") obtainable from the venom gland of a Korean viper, Salmosa (Agkistrodon halys brevicaudus), and to an amino acid sequence translated therefrom. The cDNA sequence of Halybin can be expressed in expression systems established in recombinant E.coli, yeast, baculovirus/insect cells and other animal cells. The Halybin thus produced can be used as an active ingredient of thrombolytic and hemostatic agents.

SEQUENCE LISTING

(1) GENERAL INFORMATION

(i) Applicant:

(A) Name: MOGAM BIOTECHNOLOGY RESEARCH INSTITUTE
(B) Street: 341, Pojung-Ri, Koosung-Myun,
(C) City: Yongin-Kun, Kyonggi-Do
(D) State: not applicable
(E) Country: Korea
(F) Postal Code: 449-910

(ii) Title of Invention: A NOVEL GENE CODING THROMBIN-LIKE
PROTEASE

(iii) Number of Sequences: 15

(iv) Computer-Readable Form:

(A) Medium Type:
(B) Computer:
(C) Operating System:
(D) Software:

(2) INFORMATION FOR SEQ ID NO: 1:

(i) Sequence Characteristics:

(A) Length: 123 base pairs
(B) Type: nucleic acid
(C) Strandeness: single
(D) Topology: linear

(ii) Molecular Type: cDNA to mRNA

(xi) Sequence Description: SEQ ID NO: 1:

GTG ATG GGG GGA GAC GAA TGT AAC ATA AAT GAA CAT CGT TCC 42
Val Met Gly Gly Asp Glu Cys Asn Ile Asn Glu His Arg Ser
1               5                   10

CTT GTT GTC TTG TTT AAC TCC AGC GGT TTT CTC TGC GGT GGG 84
Leu Val Val Leu Phe Asn Ser Ser Gly Phe Leu Cys Gly Gly
15                  20                  25

ACT TTG ATC AAC CAG GAA TGG GTC CTA ACC GCC GCC CA 123
Thr Leu Ile Asn Gln Glu Trp Val Leu Thr Ala Ala His
    30                  35                  40

(3) INFORMATION FOR SEQ ID NO: 2:

(i) Sequence Characteristics:

(A) Length: 41 amino acids
(B) Type: amino acid
(D) Topology: linear

(ii) Molecular Type: peptide

(xi) Sequence Description: SEQ ID NO: 2:

Val Met Gly Gly Asp Glu Cys Asn Ile Asn Glu His Arg Ser Leu
1               5                   10                  15

Val Val Leu Phe Asn Ser Ser Gly Phe Leu Cys Gly Gly Thr Leu
                20                  25                  30

Ile Asn Gln Glu Trp Val Leu Thr Ala Ala His
                35                  40

(4) INFORMATION FOR SEQ ID NO: 3:

(i) Sequence Characteristics:

(A) Length: 41 amino acids
(B) Type: amino acid
(D) Topology: linear

(ii) Molecular Type: peptide

(xi) Sequence Description: SEQ ID NO: 3:

Val Ile Gly Gly Asp Glu Cys Asp Ile Asn Glu His Pro Phe Leu
1               5                   10                  15

Ala Phe Met Tyr Tyr Ser Pro Arg Tyr Phe Cys Gly Met Thr Leu
                20                  25                  30

Ile Asn Gln Glu Trp Val Leu Thr Ala Ala His
                35                  40

(5) INFORMATION FOR SEQ ID NO: 4:

(i) Sequence Characteristics:

(A) Length: 43 amino acids
(B) Type: amino acid
(D) Topology: linear

(ii) Molecular Type: peptide

(xi) Sequence Description: SEQ ID NO: 4:

Val Ile Gly Gly Asp Glu Cys Asp Ile Asn Glu His Pro Phe Leu
1               5                   10                  15

Val Ala Leu Tyr Asp Ala Trp Ser Gly Arg Phe Leu Cys Gly Gly
                20                  25                  30

Thr Leu Ile Asn Pro Glu Trp Val Leu Thr Ala Ala His
                35                  40

(6) INFORMATION FOR SEQ ID NO: 5:

(i) Sequence Characteristics:

(A) Length: 1003 base pairs
(B) Type: nucleic acid
(C) Strandeness: single
(D) Topology: linear

(ii) Molecular Type: cDNA to mRNA

(xi) Sequence Description: SEQ ID NO: 5:

GGCACGAGGA TCTTACAGGT AAACAGCTTG CCGGCCAGAG CTGAAGCT 48

ATG GCG CTG ATC AGA GTG CTA GCA AAC CTT CTG ATA CTA CAG 90
Met Ala Leu Ile Arg Val Leu Ala Asn Leu Leu Ile Leu Gln
1               5                   10

CTT TCT TAC GAT TTG CTG CAT TAT ACA GTG TGC AAT ATC TGG 132
Leu Ser Tyr Asp Leu Leu His Tyr Thr Val Cys Asn Ile Trp
15                  20                  25

GTC ATT GCT TCT GAA ACA AAT CTC AAA ACC GCA CAA AAG TCT 174
Val Ile Ala Ser Glu Thr Asn Leu Lys Thr Ala Gln Lys Ser
    30                  35                  40

TCT GAA CTG GTC GTT GGA GGT GAT GAA TGT AAC ATA AAT GAA 216
Ser Glu Leu Val Val Gly Gly Asp Glu Cys Asn Ile Asn Glu
        45                  50                  55

CAT CGT TCC CTT GTT GTC TTG TTT AAC TCC AGC GGT TTA ATC 258
His Arg Ser Leu Val Val Leu Phe Asn Ser Ser Gly Leu Ile
            60                  65                  70

TGC AGT GGG ACT TTG ATC AAC CAG GAA TGG GTG CTC ACC GCT 300
Cys Ser Gly Thr Leu Ile Asn Gln Glu Trp Val Leu Thr Ala
                75                  80

GCA CAC TGC GAC AGT AAA AAC TTT CAG ATG CTG TTT GGT GTG 342
Ala His Cys Asp Ser Lys Asn Phe Gln Met Leu Phe Gly Val
85                  90                  95

CAT AGC AAA AAG ATA CTA AAT GAG GAT GAG CAG ACA AGA GAC 384
His Ser Lys Lys Ile Leu Asn Glu Asp Glu Gln Thr Arg Asp
    100                 105                 110

CCA AAG GAG AAG TTC ATT TGT CCC AAT AAG AAA AAA GAT GAC 426
Pro Lys Glu Lys Phe Ile Cys Pro Asn Lys Lys Lys Asp Asp
        115                 120                 125

GAA AAG GAC AAG GAC ATC ATG TTG ATC AGG CTG GAC AGT CCT     468
Glu Lys Asp Lys Asp Ile Met Leu Ile Arg Leu Asp Ser Pro
            130                     135                     140

GTT AGC AAC AGT GAA CAC ATC GCG CCT CTC AGC TTG CCT TCC     510
Val Ser Asn Ser Glu His Ile Ala Pro Leu Ser Leu Pro Ser
                145                     150

AGC TCT CCC ACT GTG GAC TCA GTT TGC CGT ATT ATG GGA TGG     552
Ser Ser Pro Thr Val Asp Ser Val Cys Arg Ile Met Gly Trp
155                     160                     165

GGC ACA ATC AAA CCT GCT GAC GAG ACT TAT CCC GAT GTC CCT     594
Gly Thr Ile Lys Pro Ala Asp Glu Thr Tyr Pro Asp Val Pro
    170                     175                     180

CAT TGT GCT AAC ATT AAC ATA CTC GAT CAT ACG GTG TGT CGA     636
His Cys Ala Asn Ile Asn Ile Leu Asp His Thr Val Cys Arg
        185                     190                     195

GCA GCT TAT CCA GTG TTG CTG GCG GGA AGC AGT ACA TTG TGT     678
Ala Ala Tyr Pro Val Leu Leu Ala Gly Ser Ser Thr Leu Cys
            200                     205                     210

GCA GGT ACC CAG CAA GGA GGC AAA GAT ACA TGT GTG GGT GAC     720
Ala Gly Thr Gln Gln Gly Gly Lys Asp Thr Cys Val Gly Asp
                215                     220

TCT GGG GGA CCC CTC ATC TGT AAT GGA CAA ATC CAG GGC ATT     762
Ser Gly Gly Pro Leu Ile Cys Asn Gly Gln Ile Gln Gly Ile
225                     230                     235

GTA TCT TGG GGG GCG CAT CCT TGT GGC CAA GGT TCT AAG CCT     804
Val Ser Trp Gly Ala His Pro Cys Gly Gln Gly Ser Lys Pro
    240                     245                     250

GGT GTC TAC ACC AAG GTC TTC GAT CAT CTT GAC TGG ATC AAG     846
Gly Val Tyr Thr Lys Val Phe Asp His Leu Asp Trp Ile Lys
        255                     260                     265

AGC ATT ATT GCA GGA AAT ACA GCT GTG ACT TGC CCC CCA TGA     888
Ser Ile Ile Ala Gly Asn Thr Ala Val Thr Cys Pro Pro
            270                     275

AAACTGTAAA TGTAAAGTGT AAATGTAACA CATTAGTACA TCTCTTCTAT     938

ATCCCTAACC ATATCCGACT GCATTGGAAT CTATTCCCAG GCATGAAGCT     988

TTTTAGACTC AAATA                                           1003

(7) INFORMATION FOR SEQ ID NO: 6:

    (i) Sequence Characteristics:

        (A) Length:                 279 amino acids
        (B) Type:                   amino acid
        (D) Topology:               linear

(ii) Molecular Type:                protein

(xi) Sequence Description: SEQ ID NO: 6:

Met Ala Leu Ile Arg Val Leu Ala Asn Leu Leu Ile Leu Gln Leu
1               5                   10                  15

Ser Tyr Asp Leu Leu His Tyr Thr Val Cys Asn Ile Trp Val Ile
                20                  25                  30

Ala Ser Glu Thr Asn Leu Lys Thr Ala Gln Lys Ser Ser Glu Leu
                35                  40                  45

Val Val Gly Gly Asp Glu Cys Asn Ile Asn Glu His Arg Ser Leu
                50                  55                  60

Val Val Leu Phe Asn Ser Ser Gly Leu Ile Cys Ser Gly Thr Leu
                65                  70                  75

Ile Asn Gln Glu Trp Val Leu Thr Ala Ala His Cys Asp Ser Lys
                80                  85                  90

Asn Phe Gln Met Leu Phe Gly Val His Ser Lys Lys Ile Leu Asn
                95                  100                 105

Glu Asp Glu Gln Thr Arg Asp Pro Lys Glu Lys Phe Ile Cys Pro
                110                 115                 120

Asn Lys Lys Lys Asp Asp Glu Lys Asp Lys Asp Ile Met Leu Ile
                125                 130                 135

Arg Leu Asp Ser Pro Val Ser Asn Ser Glu His Ile Ala Pro Leu
                140                 145                 150

Ser Leu Pro Ser Ser Ser Pro Thr Val Asp Ser Val Cys Arg Ile
                155                 160                 165

Met Gly Trp Gly Thr Ile Lys Pro Ala Asp Glu Thr Tyr Pro Asp
                170                 175                 180

Val Pro His Cys Ala Asn Ile Asn Ile Leu Asp His Thr Val Cys
                185                 190                 195

Arg Ala Ala Tyr Pro Val Leu Leu Ala Gly Ser Ser Thr Leu Cys
                200                 205                 210

Ala Gly Thr Gln Gln Gly Gly Lys Asp Thr Cys Val Gly Asp Ser
                215                 220                 225

Gly Gly Pro Leu Ile Cys Asn Gly Gln Ile Gln Gly Ile Val Ser
                230                 235                 240

Trp Gly Ala His Pro Cys Gly Gln Gly Ser Lys Pro Gly Val Tyr
                245                 250                 255

Thr Lys Val Phe Asp His Leu Asp Trp Ile Lys Ser Ile Ile Ala
                260                 265                 270

Gly Asn Thr Ala Val Thr Cys Pro Pro
                275

(8) INFORMATION FOR SEQ ID NO: 7:

   (i) Sequence Characteristics:

      (A) Length:                41 amino acids
      (B) Type:                  amino acid
      (D) Topology:              linear

  (ii) Molecular Type:           peptide

  (xi) Sequence Description: SEQ ID NO: 7:

Val Val Gly Gly Asp Glu Cys Asn Ile Asn Glu His Arg Ser
  1               5                   10

Leu Val Val Leu Phe Asn Ser Ser Gly Leu Ile Cys Ser Gly
 15                   20                  25

Thr Leu Ile Asn Gln Glu Trp Val Leu Thr Ala Ala His
     30                  35                  40

(9) INFORMATION FOR SEQ ID NO: 8:

   (i) Sequence Characteristics:

      (A) Length:                234 amino acids
      (B) Type:                  amino acid
      (D) Topology:              linear

  (ii) Molecular Type:           protein

  (xi) Sequence Description: SEQ ID NO: 8:

Val Val Gly Gly Asp Glu Cys Asn Ile Asn Glu His Arg Ser Leu
  1               5                   10                  15

Val Val Leu Phe Asn Ser Ser Gly Leu Ile Cys Ser Gly Thr Leu
                 20                  25                  30

Ile Asn Gln Glu Trp Val Leu Thr Ala Ala His Cys Asp Ser Lys
                 35                  40                  45

Asn Phe Gln Met Leu Phe Gly Val His Ser Lys Lys Ile Leu Asn
                 50                  55                  60

Glu Asp Glu Gln Thr Arg Asp Pro Lys Glu Lys Phe Ile Cys Pro
                 65                  70                  75

Asn Lys Lys Lys Asp Asp Glu Lys Asp Lys Asp Ile Met Leu Ile
                 80                  85                  90

Arg Leu Asp Ser Pro Val Ser Asn Ser Glu His Ile Ala Pro Leu
                 95                 100                 105

Ser Leu Pro Ser Ser Ser Pro Thr Val Asp Ser Val Cys Arg Ile
                110                 115                 120

Met Gly Trp Gly Thr Ile Lys Pro Ala Asp Glu Thr Tyr Pro Asp
                125                 130                 135

Val Pro His Cys Ala Asn Ile Asn Ile Leu Asp His Thr Val Cys
                140                 145                 150

Arg Ala Ala Tyr Pro Val Leu Leu Ala Gly Ser Ser Thr Leu Cys
                155                 160                 165

Ala Gly Thr Gln Gln Gly Gly Lys Asp Thr Cys Val Gly Asp Ser
                170                 175                 180

Gly Gly Pro Leu Ile Cys Asn Gly Gln Ile Gln Gly Ile Val Ser
                185                 190                 195

Trp Gly Ala His Pro Cys Gly Gln Gly Ser Lys Pro Gly Val Tyr
                200                 205                 210

Thr Lys Phe Asp His Asn Leu Asp Trp Ile Lys Ser Ile Ile Ala
                215                 220                 225

Gly Asn Thr Ala Val Thr Cys Pro Pro
                230

(10) INFORMATION FOR SEQ ID NO: 9:

(i) Sequence Characteristics:

(A) Length:                    236 amino acids
(B) Type:                      amino acid
(D) Topology:                  linear

(ii) Molecular Type:           protein

(xi) Sequence Description: SEQ ID NO: 9:

Val Ile Gly Gly Asp Glu Cys Asp Ile Asn Glu His Pro Phe Leu
  1               5                  10                  15

Val Ala Leu Tyr Asp Ala Trp Ser Gly Arg Phe Leu Cys Gly Gly
                 20                  25                  30

Thr Leu Ile Asn Pro Glu Trp Val Leu Thr Ala Ala His Cys Asp
                 35                  40                  45

Ser Lys Asn Phe Lys Met Lys Leu Gly Ala His Ser Gln Lys Val
                 50                  55                  60

Leu Asn Glu Asp Glu Gln Ile Arg Asn Pro Lys Glu Lys Phe Ile
                 65                  70                  75

Cys Pro Asn Lys Lys Asn Thr Glu Val Leu Asp Lys Asp Ile Met
                 80                  85                  90

Leu Ile Lys Leu Asp Ser Pro Val Ser Tyr Ser Glu His Ile Ala
                95                  100                 105

Pro Leu Ser Leu Pro Ser Ser Pro Pro Ser Val Gly Ser Val Cys
                110                 115                 120

Arg Ile Met Gly Trp Gly Ser Ile Thr Pro Val Glu Glu Thr Phe
                125                 130                 135

Pro Asp Val Pro His Cys Ala Asn Ile Asn Leu Leu Asp Asp Val
                140                 145                 150

Glu Cys Lys Pro Gly Tyr Pro Glu Leu Leu Pro Glu Tyr Arg Thr
                155                 160                 165

Leu Cys Ala Gly Val Leu Gln Gly Gly Ile Asp Thr Cys Gly Phe
                170                 175                 180

Asp Ser Gly Thr Pro Leu Ile Cys Asn Gly Gln Phe Gln Gly Ile
                185                 190                 195

Val Tyr Ile Gly Ser His Pro Cys Gly Gln Ser Arg Lys Pro Gly
                200                 205                 210

Ile Tyr Thr Lys Phe Asp His Tyr Asn Ala Trp Ile Gln Ser Ile
                215                 220                 225

Ile Ala Gly Asn Thr Ala Ala Thr Cys Lys Pro
                230                 235

(11) INFORMATION FOR SEQ ID NO: 10:

(i) Sequence Characteristics:

(A) Length:                232 amino acids
(B) Type:                  amino acid
(D) Topology:              linear

(ii) Molecular Type:       protein

(xi) Sequence Description: SEQ ID NO: 10:

Val Ile Gly Gly Asp Glu Cys Asp Ile Asn Glu His Pro Phe Leu
 1              5                   10                  15

Ala Phe Met Tyr Tyr Ser Pro Arg Tyr Phe Cys Gly Met Thr Leu
                20                  25                  30

Ile Asn Gln Glu Trp Val Leu Thr Ala Ala His Cys Asn Arg Arg
                35                  40                  45

Phe Met Arg Ile His Leu Gly Lys His Ala Gly Ser Val Ala Asn
                50                  55                  60

Tyr Asp Glu Val Val Arg Tyr Pro Lys Glu Lys Phe Ile Cys Pro
                65                  70                  75

Asn Lys Lys Lys Asn Val Ile Thr Asp Lys Asp Ile Met Leu Ile
                80                  85                  90

Arg Leu Asp Arg Pro Val Lys Asn Ser Glu His Ile Ala Pro Leu
                95                  100                 105

Ser Leu Pro Ser Asn Pro Pro Ser Val Gly Ser Val Cys Arg Ile
                110                 115                 120

Met Gly Trp Gly Ala Ile Thr Thr Ser Glu Asp Thr Tyr Pro Asp
                125                 130                 135

Val Pro His Cys Ala Asn Ile Asn Leu Phe Met Asn Thr Val Cys
                140                 145                 150

Arg Glu Ala Tyr Asn Gly Leu Pro Ala Lys Thr Leu Cys Ala Gly
                155                 160                 165

Val Leu Gln Gly Gly Ile Asp Thr Cys Gly Gly Asp Ser Gly Gly
                170                 175                 180

Pro Leu Ile Cys Asn Gly Gln Phe Gln Gly Ile Leu Ser Trp Gly
                185                 190                 195

Ser Asp Pro Cys Ala Glu Pro Arg Lys Pro Ala Phe Tyr Thr Lys
                200                 205                 210

Phe Asp His Tyr Leu Pro Trp Ile Gln Ser Ile Ile Ala Gly Asn
                215                 220                 225

Lys Thr Ala Thr Cys Pro Pro
                230

(12) INFORMATION FOR SEQ ID NO: 11:

(i) Sequence Characteristics:

(A) Length:                233 amino acids
(B) Type:                  amino acid
(D) Topology:              linear

(ii) Molecular Type:       protein

(xi) Sequence Description: SEQ ID NO: 11:

Val Ile Gly Gly Asp Glu Cys Asp Ile Asn Glu His Pro Phe Leu
1               5                   10                  15

Ala Phe Met Tyr Tyr Ser Pro Gln Phe Cys Gly Met Thr Leu Ile
                20                  25                  30

Asn Pro Glu Trp Val Leu Thr Ala Ala His Cys Asp Arg Thr Tyr
                35                  40                  45

Met Arg Ile Tyr Leu Gly Ile His Thr Arg Ser Val Ala Asn Asp
                50                  55                  60

Asp Glu Val Ile Arg Tyr Pro Lys Glu Lys Phe Ile Cys Pro Asn
                65                  70                  75

Lys Lys Lys Asn Val Ile Thr Asp Lys Asp Ile Met Leu Ile Arg
                80                  85                  90

Leu Asn Arg Pro Val Lys Asn Ser Thr His Ile Ala Pro Leu Ser
                95                  100                 105

Leu Pro Ser Asn Pro Pro Ser Val Gly Ser Val Cys Arg Ile Met
                110                 115                 120

Gly Trp Gly Ala Ile Thr Thr Ser Glu Asp Thr Tyr Pro Asp Val
                125                 130                 135

Pro His Cys Ala Asn Ile Asn Leu Phe Met Asn Thr Val Cys Arg
                140                 145                 150

Glu Ala Tyr Asn Gly Leu Pro Ala Gly Lys Thr Thr Leu Cys Ala
                155                 160                 165

Gly Val Leu Gln Gly Gly Ile Asp Thr Cys Gly Gly Asp Ser Gly
                170                 175                 180

Gly Pro Leu Ile Cys Asn Gly Gln Phe Gln Gly Ile Leu Ser Trp
                185                 190                 195

Gly Ser Asp Pro Cys Ala Glu Pro Arg Lys Pro Ala Phe Tyr Thr
                200                 205                 210

Lys Phe Asp His Tyr Leu Pro Trp Ile Gln Ser Ile Ile Ala Gly
                215                 220                 225

Asn Lys Thr Ala Thr Cys Pro Pro
                230

(13) INFORMATION FOR SEQ ID NO: 12:

   (i) Sequence Characteristics:

      (A) Length:                234 amino acids
      (B) Type:                  amino acid
      (D) Topology:              linear

   (ii) Molecular Type:          protein

   (xi) Sequence Description: SEQ ID NO: 12:

Val Ile Gly Gly Asp Glu Cys Asp Ile Asn Glu His Arg Phe Leu
1               5                   10                  15

Val Ala Val Tyr Glu Gly Thr Asn Trp Thr Phe Ile Cys Gly Gly
                20                  25                  30

Val Leu Ile His Pro Glu Trp Val Leu Thr Ala Glu His Cys Ala
                35                  40                  45

Arg Arg Arg Met Asn Leu Val Phe Gly Met His Arg Lys Ser Glu
                50                  55                  60

Lys Phe Asp Asp Glu Gln Glu Arg Tyr Pro Lys Lys Arg Tyr Phe
                65                  70                  75

Ile Arg Cys Asn Lys Thr Arg Thr Ser Trp Asp Glu Asp Ile Met
                80                  85                  90

Leu Ile Arg Leu Asn Lys Pro Val Asn Asn Ser Glu His Ile Ala
                95                  100                 105

Pro Leu Ser Leu Pro Ser Asn Pro Pro Ile Val Gly Ser Val Cys
                110                 115                 120

Arg Ile Met Gly Trp Gly Ser Ile Asn Arg Arg Ile Asp Val Leu
                125                 130                 135

Ser Asp Glu Pro Arg Cys Ala Asn Ile Asn Leu His Asn Phe Thr
                140                 145                 150

Met Cys His Gly Leu Phe Arg Lys Met Pro Lys Lys Gly Arg Val
                155                 160                 165

Leu Cys Ala Gly Asp Leu Arg Gly Arg Arg Asp Ser Cys Asn Ser
                170                 175                 180

Asp Ser Gly Gly Pro Leu Ile Cys Asn Glu Glu Leu His Gly Ile
                185                 190                 195

Val Ala Arg Gly Pro Asn Pro Cys Ala Gln Pro Asn Lys Pro Ala
                200                 205                 210

Leu Tyr Thr Ser Ile Tyr His Tyr Arg Asp Trp Val Asn Asn Val
                215                 220                 225

Ile Ala Gly Asn Ala Thr Cys Ser Pro
                230

(14) INFORMATION FOR SEQ ID NO: 13:

(i) Sequence Characteristics:

(A) Length: 20 base pairs
(B) Type: nucleic acid
(C) Strandeness: single
(D) Topology: linear

(xi) Sequence Description: SEQ ID NO: 13:

GTIATIGGIG GNGA(T/C)GA(A/G)TG 20

(15) INFORMATION FOR SEQ ID NO: 14:

(i) Sequence Characteristics:

(A) Length: 19 base pairs
(B) Type: nucleic acid
(C) Strandeness: single
(D) Topology: linear

(xi) Sequence Description: SEQ ID NO: 14:

TGIGCIGCIG TNA(A/G)NCCCA 19

(16) INFORMATION FOR SEQ ID NO: 15:

(i) Sequence Characteristics:

(A) Length: 31 base pairs
(B) Type: nucleic acid
(C) Strandeness: single
(D) Topology: linear

(xi) Sequence Description: SEQ ID NO: 15:

CGTTCCCTTG TTGTCTTGTT TAACTCCAGC G 31

SEQUENCE LISTING

(1) GENERAL INFORMATION:

(i) APPLICANT:
(A) NAME: MOGAM BIOTECHNOLOGY RESEARCH INSTITUTE
(B) STREET: 341, Pojung-Ri, Koosung-Myun
(C) CITY: Yongin-Kun, Kyonggi-Do
(D) STATE: not applicable
(E) COUNTRY: Korea
(F) POSTAL CODE (ZIP): 449-910
(G) TELEPHONE: 02-741-0611
(H) TELEFAX: 0331-281-6622

(ii) TITLE OF INVENTION: A NOVEL GENE CODING THROMBIN-LIKE PROTEASE

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 95305675.1

(2) INFORMATION FOR SEQ ID NO: 1:

(i) SEQUENCE CHARACTERISTICS:
(A) LENGTH: 122 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Agkistrodon halys brevicaudus
(F) TISSUE TYPE: venom gland

(vii) IMMEDIATE SOURCE:
(B) CLONE: pcr product

(ix) FEATURE:
(A) NAME/KEY: CDS
(B) LOCATION:1..122

(xi) SEQUENCE DESCRIPTION: SEQ ID NO: 1:

GTG ATG GGG GGA GAC GAA TGT AAC ATA AAT GAA CAT CGT TCC CTT GTT 48
Val Met Gly Gly Asp Glu Cys Asn Ile Asn Glu His Arg Ser Leu Val
1               5                   10                  15

GTC TTG TTT AAC TCC AGC GGT TTT CTC TGC GGT GGG ACT TTG ATC AAC 96
Val Leu Phe Asn Ser Ser Gly Phe Leu Cys Gly Gly Thr Leu Ile Asn
            20                  25                  30

CAG GAA TGG GTC CTA ACC GCC GCC CA 122
Gln Glu Trp Val Leu Thr Ala Ala His
        35                  40

(2) INFORMATION FOR SEQ ID NO: 2:

(i) SEQUENCE CHARACTERISTICS:
(A) LENGTH: 41 amino acids
(B) TYPE: amino acid
(D) TOPOLOGY: unknown

(ii) MOLECULE TYPE: peptide

(vi) ORIGINAL SOURCE:
(A) ORGANISM: Agkistrodon halys brevicaudus

(vii) IMMEDIATE SOURCE:
(B) CLONE: pcr product - aa

(ix) FEATURE:
(A) NAME/KEY: Active-site
(B) LOCATION: 41

(xi) SEQUENCE DESCRIPTION: SEQ ID NO: 2:

Val Met Gly Gly Asp Glu Cys Asn Ile Asn Glu His Arg Ser Leu Val
1               5                   10                  15

Val Leu Phe Asn Ser Ser Gly Phe Leu Cys Gly Gly Thr Leu Ile Asn
            20                  25                  30

Gln Glu Trp Val Leu Thr Ala Ala His
        35                  40

(2) INFORMATION FOR SEQ ID NO: 3:

(i) SEQUENCE CHARACTERISTICS:
(A) LENGTH: 41 amino acids
(B) TYPE: amino acid
(D) TOPOLOGY: unknown

(ii) MOLECULE TYPE: peptide

(vi) ORIGINAL SOURCE:
(A) ORGANISM: Bothrops atrox moojeni

(vii) IMMEDIATE SOURCE:
(B) CLONE: BATROXOBIN

(ix) FEATURE:
(A) NAME/KEY: Active-site

(B) LOCATION: 41

(xi) SEQUENCE DESCRIPTION: SEQ ID NO: 3:

Val Met Gly Gly Asp Glu Cys Asn Ile Asn Glu His Arg Ser Leu -Val
1               5                   10                  15

Val Leu Phe Asn Ser Ser Gly Phe Leu Cys Gly Gly Thr Leu Ile Asn
            20                  25                  30

Gln Glu Trp Val Leu Thr Ala Ala His
        35                  40

(2) INFORMATION FOR SEQ ID NO: 4:

(i) SEQUENCE CHARACTERISTICS:
(A) LENGTH: 43 amino acids
(B) TYPE: amino acid
(D) TOPOLOGY: unknown

(ii) MOLECULE TYPE: peptide

(vi) ORIGINAL SOURCE:
(A) ORGANISM: Trimeresurus flavoviridis

(vii) IMMEDIATE SOURCE:
(B) CLONE: FLAVOXOBIN

(ix) FEATURE:
(A) NAME/KEY: Active-site
(B) LOCATION: 43

(xi) SEQUENCE DESCRIPTION: SEQ ID NO: 4:

Val Ile Gly Gly Asp Glu Cys Asp Ile Asn Glu His Pro Phe Leu Val
1               5                   10                  15

Ala Leu Tyr Asp Ala Trp Ser Gly Arg Phe Leu Cys Gly Gly Thr Leu
            20                  25                  30

Ile Asn Pro Glu Trp Val Leu Thr Ala Ala His
        35                  40

(2) INFORMATION FOR SEQ ID NO: 5:

(i) SEQUENCE CHARACTERISTICS:
(A) LENGTH: 1003 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:

(A) ORGANISM: Agkistrodon halys brevicaudus

(vii) IMMEDIATE SOURCE:
(B) CLONE: protease

(ix) FEATURE:
(A) NAME/KEY: CDS
(B) LOCATION: 49..885

(ix) FEATURE:
(A) NAME/KEY: mat_peptide
(B) LOCATION: 184..885

(ix) FEATURE:
(A) NAME/KEY: sig_peptide
(B) LOCATION: 49..183

(ix) FEATURE:
(A) NAME/KEY: 5'UTR
(B) LOCATION: 1..48

(ix) FEATURE:
(A) NAME/KEY: 3'UTR
(B) LOCATION: 889..1003

(xi) SEQUENCE DESCRIPTION: SEQ ID NO: 5:

GGCACGAGGA TCTTACAGGT AAACAGCTTG CCGGCCAGAG CTGAAGCT ATG GCG CTG 57
Met Ala Leu
-45

ATC AGA GTG CTA GCA AAC CTT CTG ATA CTA CAG CTT TCT TAC GAT TTG 105
Ile Arg Val Leu Ala Asn Leu Leu Ile Leu Gln Leu Ser Tyr Asp Leu
-40 -35 -30

CTG CAT TAT ACA GTG TGC AAT ATC TGG GTC ATT GCT TCT GAA ACA AAT 153
Leu His Tyr Thr Val Cys Asn Ile Trp Val Ile Ala Ser Glu Thr Asn
-25 -20 -15

CTC AAA ACC GCA CAA AAG TCT TCT GAA CTG GTC GTT GGA GGT GAT GAA 201
Leu Lys Thr Ala Gln Lys Ser Ser Glu Leu Val Val Gly Gly Asp Glu
-10 -5 1 5

TGT AAC ATA AAT GAA CAT CGT TCC CTT GTT GTC TTG TTT AAC TCC AGC 249
Cys Asn Ile Asn Glu His Arg Ser Leu Val Val Leu Phe Asn Ser Ser
10 15 20

GGT TTA ATC TGC AGT GGG ACT TTG ATC AAC CAG GAA TGG GTG CTC ACC 297
Gly Leu Ile Cys Ser Gly Thr Leu Ile Asn Gln Glu Trp Val Leu Thr
25 30 35

GCT GCA CAC TGC GAC AGT AAA AAC TTT CAG ATG CTG TTT GGT GTG CAT 345
Ala Ala His Cys Asp Ser Lys Asn Phe Gln Met Leu Phe Gly Val His
40 45 50

AGC AAA AAG ATA CTA AAT GAG GAT GAG CAG ACA AGA GAC CCA AAG GAG 393
Ser Lys Lys Ile Leu Asn Glu Asp Glu Gln Thr Arg Asp Pro Lys Glu
55 60 65 70

AAG TTC ATT TGT CCC AAT AAG AAA AAA GAT GAC GAA AAG GAC AAG GAC 441
Lys Phe Ile Cys Pro Asn Lys Lys Lys Asp Asp Glu Lys Asp Lys Asp -
75 80 85

ATC ATG TTG ATC AGG CTG GAC AGT CCT GTT AGC AAC AGT GAA CAC ATC 489
Ile Met Leu Ile Arg Leu Asp Ser Pro Val Ser Asn Ser Glu His Ile
90 95 100

GCG CCT CTC AGC TTG CCT TCC AGC TCT CCC ACT GTG GAC TCA GTT TGC 537
Ala Pro Leu Ser Leu Pro Ser Ser Ser Pro Thr Val Asp Ser Val Cys
105 110 115

CGT ATT ATG GGA TGG GGC ACA ATC AAA CCT GCT GAC GAG ACT TAT CCC 585
Arg Ile Met Gly Trp Gly Thr Ile Lys Pro Ala Asp Glu Thr Tyr Pro
120 125 130

GAT GTC CCT CAT TGT GCT AAC ATT AAC ATA CTC GAT CAT ACG GTG TGT 633
Asp Val Pro His Cys Ala Asn Ile Asn Ile Leu Asp His Thr Val Cys
135 140 145 150

CGA GCA GCT TAT CCA GTG TTG CTG GCG GGA AGC AGT ACA TTG TGT GCA 681
Arg Ala Ala Tyr Pro Val Leu Leu Ala Gly Ser Ser Thr Leu Cys Ala
155 160 165

GGT ACC CAG CAA GGA GGC AAA GAT ACA TGT GTG GGT GAC TCT GGG GGA 729
Gly Thr Gln Gln Gly Gly Lys Asp Thr Cys Val Gly Asp Ser Gly Gly
170 175 180

CCC CTC ATC TGT AAT GGA CAA ATC CAG GGC ATT GTA TCT TGG GGG GCG 777
Pro Leu Ile Cys Asn Gly Gln Ile Gln Gly Ile Val Ser Trp Gly Ala
185 190 195

CAT CCT TGT GGC CAA GGT TCT AAG CCT GGT GTC TAC ACC AAG GTC TTC 825
His Pro Cys Gly Gln Gly Ser Lys Pro Gly Val Tyr Thr Lys Val Phe
200 205 210

GAT CAT CTT GAC TGG ATC AAG AGC ATT ATT GCA GGA AAT ACA GCT GTG 873
Asp His Leu Asp Trp Ile Lys Ser Ile Ile Ala Gly Asn Thr Ala Val
215 220 225 230

ACT TGC CCC CCA TGAAAACTGT AAATGTAAAG TGTAAATGTA ACACATTAGT 925
Thr Cys Pro Pro

ACATCTCTTC TATATCCCTA ACCATATCCG ACTGCATTGG AATCTATTCC CAGGCATGAA 985

GCTTTTTAGA CTCAAATA 1003

(2) INFORMATION FOR SEQ ID NO: 6:

(i) SEQUENCE CHARACTERISTICS:
(A) LENGTH: 279 amino acids

(B) TYPE: amino acid
(D) TOPOLOGY: unknown

(ii) MOLECULE TYPE: protein

(vi) ORIGINAL SOURCE:
(A) ORGANISM: Agkistrodon halys brevicaudus

(vii) IMMEDIATE SOURCE:
(B) CLONE: protease - aa

(xi) SEQUENCE DESCRIPTION: SEQ ID NO: 6:

Met Ala Leu Ile Arg Val Leu Ala Asn Leu Leu Ile Leu Gln Leu Ser
1               5                   10                  15

Tyr Asp Leu Leu His Tyr Thr Val Cys Asn Ile Trp Val Ile Ala Ser
            20                  25                  30

Glu Thr Asn Leu Lys Thr Ala Gln Lys Ser Ser Glu Leu Val Val Gly
        35                  40                  45

Gly Asp Glu Cys Asn Ile Asn Glu His Arg Ser Leu Val Val Leu Phe
    50                  55                  60

Asn Ser Ser Gly Leu Ile Cys Ser Gly Thr Leu Ile Asn Gln Glu Trp
65                  70                  75                  80

Val Leu Thr Ala Ala His Cys Asp Ser Lys Asn Phe Gln Met Leu Phe
                85                  90                  95

Gly Val His Ser Lys Lys Ile Leu Asn Glu Asp Glu Gln Thr Arg Asp
            100                 105                 110

Pro Lys Glu Lys Phe Ile Cys Pro Asn Lys Lys Lys Asp Asp Glu Lys
        115                 120                 125

Asp Lys Asp Ile Met Leu Ile Arg Leu Asp Ser Pro Val Ser Asn Ser
    130                 135                 140

Glu His Ile Ala Pro Leu Ser Leu Pro Ser Ser Ser Pro Thr Val Asp
145                 150                 155                 160

Ser Val Cys Arg Ile Met Gly Trp Gly Thr Ile Lys Pro Ala Asp Glu
                165                 170                 175

Thr Tyr Pro Asp Val Pro His Cys Ala Asn Ile Asn Ile Leu Asp His
            180                 185                 190

Thr Val Cys Arg Ala Ala Tyr Pro Val Leu Leu Ala Gly Ser Ser Thr
        195                 200                 205

Leu Cys Ala Gly Thr Gln Gln Gly Gly Lys Asp Thr Cys Val Gly Asp
    210                 215                 220

Ser Gly Gly Pro Leu Ile Cys Asn Gly Gln Ile Gln Gly Ile Val Ser
225                 230                 235                 240

Trp Gly Ala His Pro Cys Gly Gln Gly Ser Lys Pro Gly Val Tyr Thr
                245                 250                 255

Lys Val Phe Asp His Leu Asp Trp Ile Lys Ser Ile Ile Ala Gly Asn
            260                 265                 270

Thr Ala Val Thr Cys Pro Pro
        275

(2) INFORMATION FOR SEQ ID NO: 7:

(i) SEQUENCE CHARACTERISTICS:
(A) LENGTH: 41 amino acids
(B) TYPE: amino acid
(D) TOPOLOGY: unknown

(ii) MOLECULE TYPE: peptide

(v) FRAGMENT TYPE: N-terminal

(vi) ORIGINAL SOURCE:
(A) ORGANISM: Agkistrodon halys brevicaudus

(vii) IMMEDIATE SOURCE:
(B) CLONE: halybin peptide

(ix) FEATURE:
(A) NAME/KEY: Active-site
(B) LOCATION: 41

(xi) SEQUENCE DESCRIPTION: SEQ ID NO: 7:

Val Val Gly Gly Asp Glu Cys Asn Ile Asn Glu His Arg Ser Leu Val
1               5                   10                  15

Val Leu Phe Asn Ser Ser Gly Leu Ile Cys Ser Gly Thr Leu Ile Asn
            20                  25                  30

Gln Glu Trp Val Leu Thr Ala Ala His
        35                  40

(2) INFORMATION FOR SEQ ID NO: 8:

(i) SEQUENCE CHARACTERISTICS:
(A) LENGTH: 234 amino acids
(B) TYPE: amino acid
(D) TOPOLOGY: unknown

(ii) MOLECULE TYPE: protein

(vi) ORIGINAL SOURCE:
(A) ORGANISM: Agkistrodon halys brevicaudus

(vii) IMMEDIATE SOURCE:
(B) CLONE: aa sequence

(ix) FEATURE:
(A) NAME/KEY: Active-site
(B) LOCATION: 39

(ix) FEATURE:
(A) NAME/KEY: Active-site
(B) LOCATION: 86

(ix) FEATURE:
(A) NAME/KEY: Active-site
(B) LOCATION: 180

(xi) SEQUENCE DESCRIPTION: SEQ ID NO: 8:

Val Val Gly Gly Asp Glu Cys Asn Ile Asn Glu His Arg Ser Leu Val
1               5                   10                  15

Val Leu Phe Asn Ser Ser Gly Leu Ile Cys Ser Gly Thr Leu Ile Asn
            20                  25                  30

Gln Glu Trp Val Leu Thr Ala Ala His Cys Asp Ser Lys Asn Phe Gln
        35                  40                  45

Met Leu Phe Gly Val His Ser Lys Lys Ile Leu Asn Glu Asp Glu Gln
    50                  55                  60

Thr Arg Asp Pro Lys Glu Lys Phe Ile Cys Pro Asn Lys Lys Lys Asp
65                  70                  75                  80

Asp Glu Lys Asp Lys Asp Ile Met Leu Ile Arg Leu Asp Ser Pro Val
                85                  90                  95

Ser Asn Ser Glu His Ile Ala Pro Leu Ser Leu Pro Ser Ser Ser Pro
            100                 105                 110

Thr Val Asp Ser Val Cys Arg Ile Met Gly Trp Gly Thr Ile Lys Pro
        115                 120                 125

Ala Asp Glu Thr Tyr Pro Asp Val Pro His Cys Ala Asn Ile Asn Ile
    130                 135                 140

Leu Asp His Thr Val Cys Arg Ala Ala Tyr Pro Val Leu Leu Ala Gly
145                 150                 155                 160

Ser Ser Thr Leu Cys Ala Gly Thr Gln Gln Gly Gly Lys Asp Thr Cys
                165                 170                 175

Val Gly Asp Ser Gly Gly Pro Leu Ile Cys Asn Gly Gln Ile Gln Gly
            180                 185                 190

Ile Val Ser Trp Gly Ala His Pro Cys Gly Gln Gly Ser Lys Pro Gly
        195                 200                 205

Val Tyr Thr Lys Phe Asp His Asn Leu Asp Trp Ile Lys Ser Ile Ile
    210                 215                 220

Ala Gly Asn Thr Ala Val Thr Cys Pro Pro
225                 230

(2) INFORMATION FOR SEQ ID NO: 9:

(i) SEQUENCE CHARACTERISTICS:
(A) LENGTH: 236 amino acids
(B) TYPE: amino acid
(D) TOPOLOGY: unknown

(ii) MOLECULE TYPE: protein

(vi) ORIGINAL SOURCE:
(A) ORGANISM: Trimeresurus flavoviridis

(vii) IMMEDIATE SOURCE:
(B) CLONE: FLAVOBOXIN

(ix) FEATURE:
(A) NAME/KEY: Active-site
(B) LOCATION: 41

(ix) FEATURE:
(A) NAME/KEY: Active-site
(B) LOCATION: 88

(ix) FEATURE:
(A) NAME/KEY: Active-site
(B) LOCATION: 182

(xi) SEQUENCE DESCRIPTION: SEQ ID NO: 9:

Val Ile Gly Gly Asp Glu Cys Asp Ile Asn Glu His Pro Phe Leu Val
1               5                   10                  15

Ala Leu Tyr Asp Ala Trp Ser Gly Arg Phe Leu Cys Gly Gly Thr Leu
            20                  25                  30

Ile Asn Pro Glu Trp Val Leu Thr Ala Ala His Cys Asp Ser Lys Asn
        35                  40                  45

Phe Lys Met Lys Leu Gly Ala His Ser Gln Lys Val Leu Asn Glu Asp
    50                  55                  60

Glu Gln Ile Arg Asn Pro Lys Glu Lys Phe Ile Cys Pro Asn Lys Lys
65                  70                  75                  80

Asn Thr Glu Val Leu Asp Lys Asp Ile Met Leu Ile Lys Leu Asp Ser
                85                  90                  95

Pro Val Ser Tyr Ser Glu His Ile Ala Pro Leu Ser Leu Pro Ser Ser
            100                 105                 110

Pro Pro Ser Val Gly Ser Val Cys Arg Ile Met Gly Trp Gly Ser Ile
        115                 120                 125

Thr Pro Val Glu Glu Thr Phe Pro Asp Val Pro His Cys Ala Asn Ile
    130                 135                 140

Asn Leu Leu Asp Asp Val Glu Cys Lys Pro Gly Tyr Pro Glu Leu Leu
145                 150                 155                 160

Pro Glu Tyr Arg Thr Leu Cys Ala Gly Val Leu Gln Gly Gly Ile Asp
                165                 170                 175

Thr Cys Gly Phe Asp Ser Gly Thr Pro Leu Ile Cys Asn Gly Gln Phe
            180                 185                 190

Gln Gly Ile Val Tyr Ile Gly Ser His Pro Cys Gly Gln Ser Arg Lys
        195                 200                 205

Pro Gly Ile Tyr Thr Lys Phe Asp His Tyr Asn Ala Trp Ile Gln Ser
    210                 215                 220

Ile Ile Ala Gly Asn Thr Ala Ala Thr Cys Lys Pro
225                 230                 235

(2) INFORMATION FOR SEQ ID NO: 10:

     (i) SEQUENCE CHARACTERISTICS:
          (A) LENGTH: 232 amino acids
          (B) TYPE: amino acid
          (D) TOPOLOGY: unknown

    (ii) MOLECULE TYPE: protein

    (vi) ORIGINAL SOURCE:
          (A) ORGANISM: Bothrops atrox moojeni

   (vii) IMMEDIATE SOURCE:
          (B) CLONE: BATROXOBIN

    (ix) FEATURE:
          (A) NAME/KEY: Active-site
          (B) LOCATION: 39

    (ix) FEATURE:
          (A) NAME/KEY: Active-site
          (B) LOCATION: 86

    (ix) FEATURE:
          (A) NAME/KEY: Active-site
          (B) LOCATION: 178

    (xi) SEQUENCE DESCRIPTION: SEQ ID NO: 10:

Val Ile Gly Gly Asp Glu Cys Asp Ile Asn Glu His Pro Phe Leu Ala
1               5                   10                  15

Phe Met Tyr Tyr Ser Pro Arg Tyr Phe Cys Gly Met Thr Leu Ile Asn
            20                  25                  30

Gln Glu Trp Val Leu Thr Ala Ala His Cys Asn Arg Arg Phe Met Arg
35                  40                  45

Ile His Leu Gly Lys His Ala Gly Ser Val Ala Asn Tyr Asp Glu Val
50                  55                  60

Val Arg Tyr Pro Lys Glu Lys Phe Ile Cys Pro Asn Lys Lys Lys Asn
65                  70                  75                  80

Val Ile Thr Asp Lys Asp Ile Met Leu Ile Arg Leu Asp Arg Pro Val
                85                  90                  95

Lys Asn Ser Glu His Ile Ala Pro Leu Ser Leu Pro Ser Asn Pro Pro
            100                 105                 110

Ser Val Gly Ser Val Cys Arg Ile Met Gly Trp Gly Ala Ile Thr Thr
        115                 120                 125

Ser Glu Asp Thr Tyr Pro Asp Val Pro His Cys Ala Asn Ile Asn Leu
    130                 135                 140

Phe Met Asn Thr Val Cys Arg Glu Ala Tyr Asn Gly Leu Pro Ala Lys
145                 150                 155                 160

Thr Leu Cys Ala Gly Val Leu Gln Gly Gly Ile Asp Thr Cys Gly Gly
                165                 170                 175

Asp Ser Gly Gly Pro Leu Ile Cys Asn Gly Gln Phe Gln Gly Ile Leu
            180                 185                 190

Ser Trp Gly Ser Asp Pro Cys Ala Glu Pro Arg Lys Pro Ala Phe Tyr
        195                 200                 205

Thr Lys Phe Asp His Tyr Leu Pro Trp Ile Gln Ser Ile Ile Ala Gly
    210                 215                 220

Asn Lys Thr Ala Thr Cys Pro Pro
225                 230

(2) INFORMATION FOR SEQ ID NO: 11:

(i) SEQUENCE CHARACTERISTICS:
(A) LENGTH: 233 amino acids
(B) TYPE: amino acid
(D) TOPOLOGY: unknown

(ii) MOLECULE TYPE: protein

(vi) ORIGINAL SOURCE:
(A) ORGANISM: Bothrops jararaca

(vii) IMMEDIATE SOURCE:
(B) CLONE: BOTHROMBIN

(ix) FEATURE:
(A) NAME/KEY: Active-site

(B) LOCATION: 38

(ix) FEATURE:
(A) NAME/KEY: Active-site
(B) LOCATION: 85

(ix) FEATURE:
(A) NAME/KEY: Active-site
(B) LOCATION: 179

(xi) SEQUENCE DESCRIPTION: SEQ ID NO: 11:

Val Ile Gly Gly Asp Glu Cys Asp Ile Asn Glu His Pro Phe Leu Ala
1               5                   10                  15

Phe Met Tyr Tyr Ser Pro Gln Phe Cys Gly Met Thr Leu Ile Asn Pro
            20                  25                  30

Glu Trp Val Leu Thr Ala Ala His Cys Asp Arg Thr Tyr Met Arg Ile
        35                  40                  45

Tyr Leu Gly Ile His Thr Arg Ser Val Ala Asn Asp Asp Glu Val Ile
    50                  55                  60

Arg Tyr Pro Lys Glu Lys Phe Ile Cys Pro Asn Lys Lys Lys Asn Val
65                  70                  75                  80

Ile Thr Asp Lys Asp Ile Met Leu Ile Arg Leu Asn Arg Pro Val Lys
                85                  90                  95

Asn Ser Thr His Ile Ala Pro Leu Ser Leu Pro Ser Asn Pro Pro Ser
            100                 105                 110

Val Gly Ser Val Cys Arg Ile Met Gly Trp Gly Ala Ile Thr Thr Ser
        115                 120                 125

Glu Asp Thr Tyr Pro Asp Val Pro His Cys Ala Asn Ile Asn Leu Phe
    130                 135                 140

Met Asn Thr Val Cys Arg Glu Ala Tyr Asn Gly Leu Pro Ala Gly Lys
145                 150                 155                 160

Thr Thr Leu Cys Ala Gly Val Leu Gln Gly Gly Ile Asp Thr Cys Gly
                165                 170                 175

Gly Asp Ser Gly Gly Pro Leu Ile Cys Asn Gly Gln Phe Gln Gly Ile
            180                 185                 190

Leu Ser Trp Gly Ser Asp Pro Cys Ala Glu Pro Arg Lys Pro Ala Phe
        195                 200                 205

Tyr Thr Lys Phe Asp His Tyr Leu Pro Trp Ile Gln Ser Ile Ile Ala
    210                 215                 220

Gly Asn Lys Thr Ala Thr Cys Pro Pro
225                 230

(2) INFORMATION FOR SEQ ID NO: 12:

(i) SEQUENCE CHARACTERISTICS:
(A) LENGTH: 234 amino acids
(B) TYPE: amino acid
(D) TOPOLOGY: unknown

(ii) MOLECULE TYPE: protein

(vi) ORIGINAL SOURCE:
(A) ORGANISM: Calloselasma rhodostoma

(vii) IMMEDIATE SOURCE:
(B) CLONE: ANCROD

(ix) FEATURE:
(A) NAME/KEY: Active-site
(B) LOCATION: 38

(ix) FEATURE:
(A) NAME/KEY: Active-site
(B) LOCATION: 88

(ix) FEATURE:
(A) NAME/KEY: Active-site
(B) LOCATION: 182

(xi) SEQUENCE DESCRIPTION: SEQ ID NO: 12:

Val Ile Gly Gly Asp Glu Cys Asp Ile Asn Glu His Arg Phe Leu Val
1               5                   10                  15

Ala Val Tyr Glu Gly Thr Asn Trp Thr Phe Ile Cys Gly Gly Val Leu
            20                  25                  30

Ile His Pro Glu Trp Val Leu Thr Ala Glu His Cys Ala Arg Arg Arg
        35                  40                  45

Met Asn Leu Val Phe Gly Met His Arg Lys Ser Glu Lys Phe Asp Asp
    50                  55                  60

Glu Gln Glu Arg Tyr Pro Lys Lys Arg Tyr Phe Ile Arg Cys Asn Lys
65                  70                  75                  80

Thr Arg Thr Ser Trp Asp Glu Asp Ile Met Leu Ile Arg Leu Asn Lys
                85                  90                  95

Pro Val Asn Asn Ser Glu His Ile Ala Pro Leu Ser Leu Pro Ser Asn
            100                 105                 110

Pro Pro Ile Val Gly Ser Val Cys Arg Ile Met Gly Trp Gly Ser Ile
        115                 120                 125

Asn Arg Arg Ile Asp Val Leu Ser Asp Glu Pro Arg Cys Ala Asn Ile
    130                 135                 140

Asn Leu His Asn Phe Thr Met Cys His Gly Leu Phe Arg Lys Met Pro
145                 150                 155                 160

Lys Lys Gly Arg Val Leu Cys Ala Gly Asp Leu Arg Gly Arg Arg Asp
                165                 170                 175

Ser Cys Asn Ser Asp Ser Gly Gly Pro Leu Ile Cys Asn Glu Glu Leu
            180                 185                 190

His Gly Ile Val Ala Arg Gly Pro Asn Pro Cys Ala Gln Pro Asn Lys
        195                 200                 205

Pro Ala Leu Tyr Thr Ser Ile Tyr His Tyr Arg Asp Trp Val Asn Asn
    210                 215                 220

Val Ile Ala Gly Asn Ala Thr Cys Ser Pro
225                 230

(2) INFORMATION FOR SEQ ID NO: 13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA to mRNA

(vii) IMMEDIATE SOURCE:
(B) CLONE: 5' PCR PRIMER

(ix) FEATURE:
(A) NAME/KEY: modified_base
(B) LOCATION: one-of(3, 6, 9)
(C) IDENTIFICATION METHOD: experimental
(D) OTHER INFORMATION:/evidence= EXPERIMENTAL
/frequency= 0.15
/mod_base= i
/label= N

(xi) SEQUENCE DESCRIPTION: SEQ ID NO: 13:

GTNATNGGNG GNGAYGARTG                                                20

(2) INFORMATION FOR SEQ ID NO: 14:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA to mRNA

(vii) IMMEDIATE SOURCE:

(B) CLONE: 3' PCR PRIMER

(ix) FEATURE:
(A) NAME/KEY: modified_base
(B) LOCATION:one-of(3, 6, 9)
(C) IDENTIFICATION METHOD: experimental
(D) OTHER INFORMATION:/evidence= EXPERIMENTAL
/frequency= 0.158
/mod_base= i
/label= N

(xi) SEQUENCE DESCRIPTION: SEQ ID NO: 14:

TGNGCNGCNG TNARNCCCA 19

(2) INFORMATION FOR SEQ ID NO: 15:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Agkistrodon halys brevicaudus

(vii) IMMEDIATE SOURCE:
(B) CLONE: mid-domain pcr

(xi) SEQUENCE DESCRIPTION: SEQ ID NO: 15:

CGTTCCCTTG TTGTCTTGTT TAACTCCAGC G 31

## Description

### Field of the Invention

The present invention relates to a novel gene coding a protease, more specifically, a novel cDNA sequence of a thrombin-like serine protease originated from the venom gland of a Korean viper, Salmosa(Agkistrodon halys brevicaudus) and an amino acid sequence translated therefrom.

### Background of the Invention

Thrombosis and hemostasis has been a principal subject in the medical field for long time. In this regard, studies on the thrombolytic agents such as urokinase, streptokinase and tissue-type plasminogen activator, have been actively carried out, and their use as therapeutic agents for thrombosis has been also practiced(see: Lijnen, H.R. et al., Thromb. Haemost., 66:88(1991)). Recently, a variety of biologically active materials have been isolated from the natural sources such as leeches, vampire bats and snake venoms, and applied in the control of thrombosis(see: Sawyer, R.T., Bio/Technol., 9:513 (1991); USP 4,568,545; Gardell, S.J. et al., J. Biol. Chem., 264:17947(1989); Meier, J. et al., Toxicology, 21:171(1991)).

Under the circumstances, snake venoms which are known to influence the hemostatic system in human, has been also investigated, and a number of materials that have an effect on the hemostatic system have been isolated from the venom and clinically tested. Among these materials, many of which have been practically introduced in basic research or in diagnosis and treatment, in particular, a variety of thrombin-like proteases isolated from snake venoms which cleave fibrinopeptide of fibrinogen to produce fibrin, i.e., thrombin-like protease, have been reported(see: Meier, J. et al., Toxicology, 21:171(1991)). To the present, 23 enzymes of this category have been identified and the amino acid sequences of four enzymes have been determined.

Among the proteases whose enzymatic characteristics are clarified, batroxobin, a thrombin-like protease isolated from the venom of "Lance head snake" (Bothrops atrox moojeni), was reported to cleave fibrinopeptide A of fibrinogen in a specific manner. A small amount of batroxobin is able to convert fibrinogen to fibrin I(Des-A fibrin) which is degraded by plasmin rapidly, thus resulting in a decrease of fibrinogen level in blood. Generally, it can induce the synthesis of plasminogen activator and accelerate degradation of thrombus, but rather causes blood clotting when a high level of batroxobin is applied. Grounded on the facts, batroxobin has been developed both as a defibrinogenating drug (commercial name "Defibrase") and as a hemostatic agent (commercial name "Reptilase")(see: Meier, J. et al., Medical Use of Snake Venom proteins, CRC Press, 136(1990)).

On the other hand, 14 species of snake have been identified in Korea, among which only three species of Agkistrodon genus are known to have venoms(see: Nah, K.Y. et al., J. Surgery, 17:13(1975); Gloyd, H.K., Proc. Biol. Soc., Washington, 85:577(1971)). Recently, from the venom of a Korean viper, Salmosa(Agkistrodon halys brevicaudus), a thrombin-like proteolytic enzyme of 39,200 dalton has been isolated, which is a glycoprotein composed of 323 amino acids and contains a high quantity of glutamic acid and aspartic acid, while biochemistry of the enzyme has not been elucidated(see: Sun, J. et al., Zhongguo Yike Daxue Xuebao, 16:276(1987); Chem. Abstr., 108:90629n(1988)). Further, it has been also reported that a 33,000 dalton and a 51,000 dalton of fibrinolytic serine proteases have been isolated from the venom of Salmosa(see: Chung, K.H. et al., Thromb. Haemost. THHADQ, 65:953(1991)).

### Summary of the Invention

In accordance with the present invention, the inventors isolated a cDNA of thrombin-like protease(hereinafter referred to as "Halybin") from the cDNA library of the venom of Salmosa, and determined an amino acid sequence translated from the cDNA.

A primary object of the present invention is, therefore, to provide a novel cDNA sequence of thrombin-like protease originated from the venom gland of a Korean viper, Salmosa(Agkistrodon halys brevicaudus).

The other object of the invention is to provide an amino acid sequence of the thrombin-like protease translated from the cDNA sequence.

### Brief Description of the Invention

The above and the other objects and features of the present invention will become apparent from the following description given in conjunction with the accompanying drawings, in which:
- Figure 1: is a schematic diagram showing the process of construction of a cDNA library of the venom gland of Salmosa;
- Figure 2A: shows DNA sequence of PCR product(SEQ ID NO:1) from the cDNA library and amino acid sequence translated therefrom(SEQ ID NO:2);
- Figure 2B: shows a comparison between amino acid sequences from the PCR product(SEQ ID NO:2) and known snake venom serine proteases(SEQ ID NO:3, SEQ ID NO:4);
- Figure 3A: is a schematic diagram showing the DNA sequence of Halybin;
- Figure 3B: shows cDNA sequence of Halybin(SEQ ID NO:5);
- Figure 3C: shows amino acid sequence(SEQ ID NO:6) translated from the cDNA of Halybin disclosed in Fig. 3B;
- Figure 4: shows a comparison between the amino acid sequences translated from the DNA sequence of PCR product and of Halybin(SEQ ID NO:2, SEQ ID NO:7); and,
- Figure 5: shows a comparison between amino acid sequences of Halybin and of snake venom serine proteases known in the art(SEQ ID NO:8, SEQ ID NO:9, SEQ ID NO:10, SEQ ID NO:11, SEQ ID NO:12).

### Detailed Description of the Invention

A cDNA of 2.1 kb encoding a thrombin-like protease was cloned, by employing an oligonucleotide probe for the non-conserved amino acids in the PCR products prepared with oligonucleotide primers for the amino acid sequences well conserved in the serine proteases isolated from the snake venom. cDNA thus cloned was determined to contain an open reading frame of 837 nucleotides encoding 279 amino acids, where the deduced amino acid sequence was composed of a mature serine protease of 234 amino acids and a signal peptide of 45 amino acids.

Presumed from its amino acid sequence, Halybin was determined to have a molecular weight of 25.7 kDa and the isoelectric point(pI) of 5.68, and was suggested as a glycoprotein of higher molecular weight in the venom, since it contains an N-glycosylation site. From the information retrieved on the proteins, it has been determined that: Halybin has 64-71 % homology in terms of amino acid sequence, in a relation to thrombin-like proteases known in the art; and, the amino acid sequences of the active site, e.g., His, Asp, and Ser and in the vicinity of the active site as well as in the N-terminal sequence, are well conserved, which is common phenomena in the serine proteases originated from the snake venom. In addition, on the basis of the finding that the protease of the present invention retains 12 cysteines, which are also well conserved throughout this category of enzymes, Halybin was suggested to have a thrombin-like protease activity similar to known proteases.

cDNA sequence of the Halybin originated from the venom of a Korean viper, Salmosa(Agkistrodon halys brevicaudus), can be expressed in proper expression systems established in recombinant E. coli, yeast, baculovirus/insect cells and other animal cells. The recombinant Halybin thus expressed can be applied as an active ingredient of thrombolytic and hemostatic agents.

In describing the amino acid sequence of protein in the present invention, one-letter symbols abbreviated by the IUPACIUB standard are employed as followings:

| Amino acid | Symbol |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

The present invention is further illustrated in the following examples, which should not be taken to limit the scope of the invention.

### Example 1: Construction of cDNA library from the venom gland of Salmosa

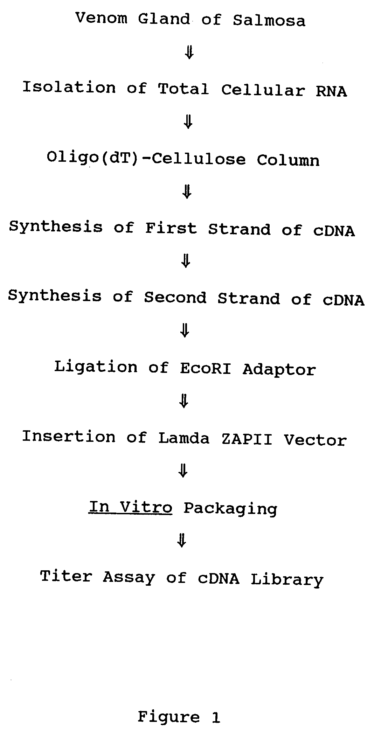

Venom gland was obtained from a Korean viper, Salmosa(Agkistrodon halys brevicaudus). As the venom gland is a pea-like tiny tissue, at least five glands were used to obtain a sufficient amount of RNA. To isolate total cellular RNA, guanidine isothiocyanate was treated, and an ultracentrifugation with CsCl cushion was followed. Poly(A)⁺RNA was separated from the total cellular RNA, by applying on an oligo(dT)-cellulose column twice. Then, first and second strands of cDNA were synthesized by employing reverse transcriptase, RNase H, and E. coli DNA polymerase I(see: Sambrook, J. et al., Molecular Cloning, 2nd Ed., Cold Spring Harbor Laboratory, 1989). Ligation of an appropriate adaptor to the cDNA thus synthesized, insertion into lamda ZAPII(Stratagene, USA), and in vitro packaging were successively carried out to construct cDNA library. cDNA library thus constructed was determined to contain 10⁶ of independent plaques. The construction process of the cDNA library from the venom gland of Salmosa was schematically depicted in Fig. 1.

### Example 2: Preparation of probe

To prepare a probe for the screening of cDNA encoding thrombin-like protease from the cDNA library constructed in Example 1, oligonucleotide primers for the well conserved amino acids in serine proteases from the snake venom were first selected as follows: Based on the amino acid sequence common to the N-terminal region of serine proteases from the snake venom, i.e., VIGGDEC, a degenerated oligonucleotide containing the following sequence was employed as 5' primer:
5' GTIATIGGIGGNGA(T/C)GA(A/G)TG 3'(SEQ ID NO:13).
Further, based on the amino acid sequence in the vicinity of histidine which is one of amino acid residues constituting the active site of serine proteases, i.e., WVLTAAH, a degenerated oligonucleotide containing the following sequence was employed as 3' primer:
5' TGIGCIGCIGTNA(A/G)NCCCA 3'(SEQ ID NO:14)
wherein,
I represents inosine; and,
N represents one of four nucleotides,
i.e., A, G, C or T.

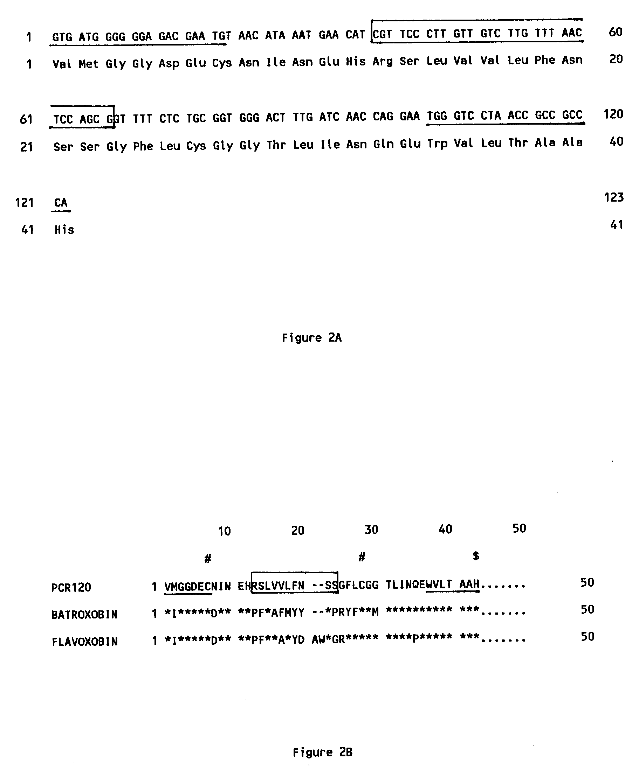

By employing the said primers, Taq DNA polymerase and the cDNA library prepared in Example 1 as a template, 25 rounds of polymerase chain reaction(PCR) was carried out for one minute at 95 °C, for two minutes at 48 °C, and for one minute at 72 °C, respectively, to obtain a PCR product of 120 bp. PCR product thus obtained was subcloned in plasmid pCRII (Invitrogen, USA), and the DNA sequence was determined(see: Fig. 2A).

The amino acid sequence derived from the DNA sequence ("PCR120") displayed 65-72 % homology with known serine proteases such as BATROXOBIN isolated from Bothrops atrox moojeni and FLAVOXOBIN isolated from Trimeresurus flavoviridis (see: Fig. 2B). In Figs. 2A and 2B, the underlined and the boxed represent DNA sequence derived from the PCR primers and DNA sequence employed in the screening of cDNA library, and amino acid sequences translated therefrom, respectively; and, (*), ($) and (#) represent conserved amino acids, an amino acid residue constituting the active site among the conserved sequence and conserved cysteine residues, respectively. The DNA sequence corresponding to the mid-domain of the PCR product of 120 bp, i.e., 5'-CGTTCCCTTGTT- GTCTTGTTTAACTCCAGCG-3ʼ (SEQ ID NO:15), was employed as a probe for the screening of cDNA library described below.

### Example 3: Screening of cDNA library

cDNA library prepared in Example 1 was plated on E. coli XL-1 Blue to obtain plaques of 1.2 X 10⁵. After transferring the plaques into a nitrocellulose membrane twice, the membrane was treated with a degenerating solution(0.5 N NaOH, 1.5 M NaCl) for 2 minutes, with a neutralizing solution(0.5 M Tris HCl(pH 7.4) containing 1.5 M NaCl) for 5 minutes, and washed in 2 X SSC for 30 seconds. After the membrane was subjected to a vacuum condition at 80 °C for two hours, prehybridization was carried out at 68 °C for 3 hours, in a solution containing 5 X SSC, 20 mM sodium phosphate(pH 6.5), 3 % SDS and 100 µg/ml of salmon sperm DNA. The probe selected in Example 2 was end-labelled with [γ-³²P]ATP, added in a concentration of 1 X 10⁶ cpm/ml to the prehybridization solution, and left to hybridize at 53 °C for 16 hours. Then, the membrane was washed with a solution containing 3 X SSC, 3 % SDS and 10 mM sodium phosphate(pH 6.5) at 53 °C for 1 hr. The membrane was exposed to an X-ray film(Agfa curix, Germany) under an intensifying screen at -70 °C for 24 hours. As a result, 11 positive clones whose spots were formed at the same position, were detected on the duplicate of the X-ray film. Hybridization of phage plaques transferred from a plate to two separate sheets of nitrocellulose membrane and exposure on the X-ray film, reveals a positive result that spots are found at the same position on each membrane. Plating with a low dilution factor and repeating secondary and tertiary screening described above, was carried out to obtain each individual plaque.

Each clone was cloned in a pBluescript vector(Stratagene, USA) and treated with a restriction enzyme, and it was elucidated that the size of the insert ranged from 0.5 kb to 2.1 kb. The sequence analysis of each clone proved that 11 clones share common sequence of a gene, and among them the full sequence of the longest clone of 2.1 kb was determined.

### Example 4: Determination of DNA sequence

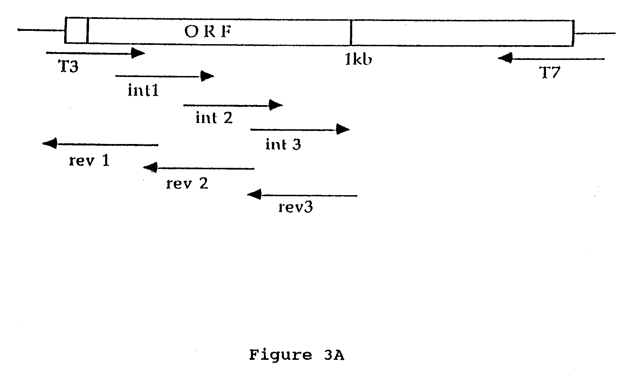

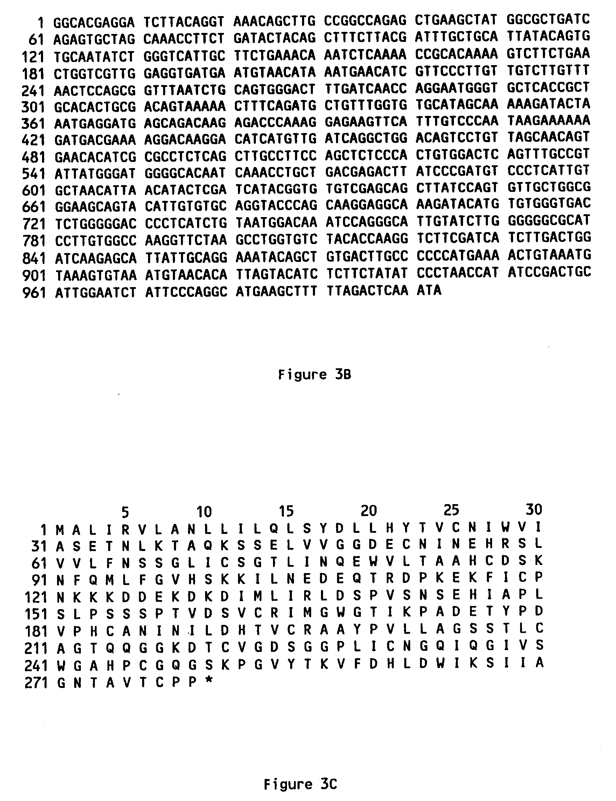

Using primers derived from the vector and the DNA sequence, respectively, DNA sequence of 2.1 kb clone was determined by the dideoxy termination method(see: Sambrook, J. et al., Molecular Cloning, 2nd Ed., 1989) employing Sequenase(USB, USA). Careful determination of the sequence was carried out over a region of the total 2.1 kb of insert containing the total open reading frame. As a result, 1003 bp of DNA sequence was analyzed, which contains 5'-untranslated region of 49 bp, ORF(open reading frame) of 837 bp and 3'-untranslated region of 114 bp(see: Fig. 3A).

### Example 5: Analysis of DNA sequence

Determination of DNA sequence revealed that the cDNA contains an open reading frame of 837 nucleotides encoding 279 amino acids. 279 amino acids were also determined to contain a signal peptide of 45 amino acids and a mature enzyme of 234 amino acids(see: Fig. 3B). Presumed from the amino acid sequence and composition, the mature enzyme was determined to be a protein of molecular weight of 25.7 kDa with the isoelectric point(pI) of 5.68, and was suggested to be a glycoprotein of higher molecular weight in the venom, since it contains an N-glycosylation site.

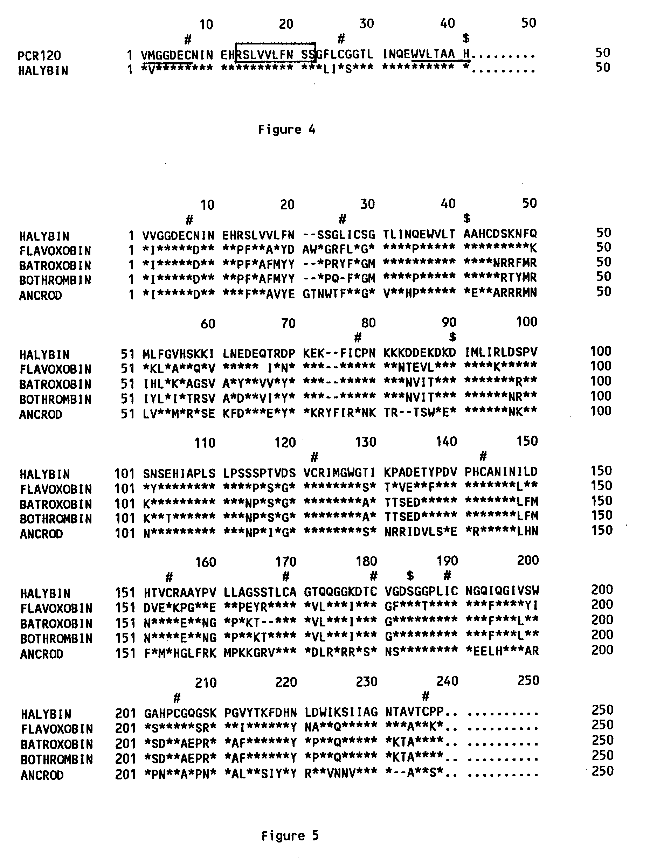

Comparison of DNA and amino acid sequences of Halybin and PCR product of 120 bp(PCR 120) revealed that three nucleotides and corresponding three amino acids are different from each other(see: Fig. 4). From the result, it is suggested that similar serine protease with a slightly different amino acid sequence exists in the venom of Salmosa. In Fig. 4, the underlined and the boxed represent amino acid sequence derived from the degenerate PCR primers and from the DNA sequence employed in the screening of cDNA library; and, (*), ($) and (#) represent conserved amino acids, an amino acid residue constituting the active site among the conserved sequence and conserved cysteine residues, respectively.

On the other hand, from the information on proteins retrieved using Swiss prot(HITACHI Software Eng. Co., USA), it was determined that Halybin displays 64-71 % homology in terms of amino acid sequence, in a relation to prior art thrombinlike proteases of snake venom(see: Fig. 5). Moreover, it was determined that Halybin contains amino acid sequences constituting the active site of common serine proteases, i.e., His, Asp, and Ser and the vicinity of the active site as well as the N-terminal sequence, which are well conserved in prior art serine proteases, e.g., FLAVOXOBIN(see: Shieh, T.-C. et al., J. Biochem.(Tokyo), 103:596-605(1988)), BATROXOBIN(see: Itoh, N. et al., J. Biol. Chem., 263:7628-7631(1988)), BOTHROMBIN(see: Nishida, S. et al., Biochemistry, 33:1843-1849(1994)) and ANCROD(see: Hatton, M.W.C., Biochem. J., 131:799-807(1973)). In addition, Halybin was determined to contain 12 cysteines, which are also well conserved throughout this category of enzymes(represented as '#' in Fig. 5).

As clearly shown and explained above, the present invention provides a cDNA sequence of a thrombin-like protease ("Halybin") originated from the venom gland of a Korean viper, Salmosa (Agkistrodon halys brevicaudus), and an amino acid sequence translated therefrom. The cDNA sequence of Halybin can be expressed in proper expression systems established in recombinant E. coli, yeast, baculovirus/insect cells and other animal cells, and Halybin thus produced can be applied as an active ingredient of thrombolytic and hemostatic agents.

## Claims

1. A cDNA encoding a thrombin-like protease (Halybin) of sequence (SEQ ID NO:5):

1 GGCACGAGGA TCTTACAGGT AAACAGCTTG CCGGCCAGAG CTGAAGCTAT GGCGCTGATC
61 AGAGTGCTAG CAAACCTTCT GATACTACAG CTTTCTTACG ATTTGCTGCA TTATACAGTG
121 TGCAATATCT GGGTCATTGC TTCTGAAACA AATCTCAAAA CCGCACAAAA GTCTTCTGAA
181 CTGGTCGTTG GAGGTGATGA ATGTAACATA AATGAACATC GTTCCCTTGT TGTCTTGTTT
241 AACTCCAGCG GTTTAATCTG CAGTGGGACT TTGATCAACC AGGAATGGGT GCTCACCGCT
301 GCACACTGCG ACAGTAAAAA CTTTCAGATG CTGTTTGGTG TGCATAGCAA AAAGATACTA
361 AATGAGGATG AGCAGACAAG AGACCCAAAG GAGAAGTTCA TTTGTCCCAA TAAGAAAAAA
421 GATGACGAAA AGGACAAGGA CATCATGTTG ATCAGGCTGG ACAGTCCTGT TAGCAACAGT
481 GAACACATCG CGCCTCTCAG CTTGCCTTCC AGCTCTCCCA CTGTGGACTC AGTTTGCCGT
541 ATTATGGGAT GGGGCACAAT CAAACCTGCT GACGAGACTT ATCCCGATGT CCCTCATTGT
601 GCTAACATTA ACATACTCGA TCATACGGTG TGTCGAGCAG CTTATCCAGT GTTGCTGGCG
661 GGAAGCAGTA CATTGTGTGC AGGTACCCAG CAAGGAGGCA AAGATACATG TGTGGGTGAC
721 TCTGGGGGAC CCCTCATCTG TAATGGACAA ATCCAGGGCA TTGTATCTTG GGGGGCGCAT
781 CCTTGTGGCC AAGGTTCTAA GCCTGGTGTC TACACCAAGG TCTTCGATCA TCTTGACTGG
841 ATCAAGAGCA TTATTGCAGG AAATACAGCT GTGACTTGCC CCCCATGAAA ACTGTAAATG
901 TAAAGTGTAA ATGTAACACA TTAGTACATC TCTTCTATAT CCCTAACCAT ATCCGACTGC
961 ATTGGAATCT ATTCCCAGGC ATGAAGCTTT TTAGACTCAA ATA.

2. A cDNA according to claim 1 which is obtainable from the venom gland of Salmosa (Agkistrodonai halys brevicaudus).

3. A probe for screening a cDNA library to identify the cDNA of claim 1 or 2, whose sequence is (SEQ ID NO: 15):
5' CGTTCCCTTGTTGTCTTGTTTAACTCCAGCG 3'.

4. An amino acid sequence corresponding to the cDNA of claim 1 or 2, which sequence is (SEQ ID NO:6):

5 10 15 20 25 30
1 M A L I R V L A N L L I L Q L S Y D L L H Y T V C N I W V I
31 A S E T N L K T A Q K S S E L V V G G D E C N I N E H R S L
61 V V L F N S S G L I C S G T L I N Q E W V L T A A H C D S K
91 N F Q M L F G V H S K K I L N E D E Q T R D P K E K F I C P
121 N K K K D D E K D K D I M L I R L D S P V S N S E H I A P L
151 S L P S S S P T V D S V C R I M G W G T I K P A D E T Y P D
181 V P H C A N I N I L D H T V C R A A Y P V L L A G S S T L C
211 A G T Q Q G G K D T C V G D S G G P L I C N G Q I Q G I V S
241 W G A H P C G Q G S K P G V Y T K V F D H L D W I K S I I A
271 G N T A V T C P P *

5. A thrombin-like protease (Halybin) obtainable from the venom of Salmosa (Agkistrodon halys brevicaudus) containing the amino acid sequence of claim 4.

6. A thrombin-like protease containing the amino acid sequence of claim 4 for use in treatment of the human or animal body by therapy.

7. A protease according to claim 6 for use as a thrombolytic or hemostatic agent.

8. A pharmaceutical composition comprising a pharmaceutically acceptable carrier or diluent and, as active ingredient, a thrombin-like protease as claimed in any one of claims 4 to 7.
